**Europäisches Patentamt**

**European Patent Office**

**Office européen des brevets**

(19)

(11) Veröffentlichungsnummer : **0 237 520 B1**

# (12) EUROPÄISCHE PATENTSCHRIFT

(45) Veröffentlichungstag der Patentschrift :
27.02.91 Patentblatt 91/09

(51) Int. Cl.⁵ : **C12P 7/06, C12F 3/10, C12F 1/00**

(21) Anmeldenummer : **87890042.2**

(22) Anmeldetag : **05.03.87**

(54) **Verfahren zur Herstellung von Äthanol aus zuckerhältigen Rohstoffen sowie Anlage zur Durchführung des Verfahrens.**

(30) Priorität : **10.03.86 AT 611/86**

(43) Veröffentlichungstag der Anmeldung :
**16.09.87 Patentblatt 87/38**

(45) Bekanntmachung des Hinweises auf die
Patenterteilung :
**27.02.91 Patentblatt 91/09**

(84) Benannte Vertragsstaaten :
**BE CH DE ES FR GB IT LI SE**

(56) Entgegenhaltungen :
**EP-A- 0 072 045**
**EP-A- 0 185 010**
**DE-A- 2 919 518**

(73) Patentinhaber : **VOGELBUSCH GESELLSCHAFT m.b.H.**
**Blechturmgasse 11**
**A-1050 Wien (AT)**

(72) Erfinder : **Gutschireiter, Friedrich**
**Neustiftgasse 16**
**A-2433 Margarethen/Moos (AT)**

(74) Vertreter : **Wolfram, Gustav, Dipl.-Ing.**
**Schwindgasse 7 P.O. Box 205**
**A-1041 Wien (AT)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Äthanol aus zuckerhältigen Rohstoffen durch gegenstrom- Extraktion der Rohstoffe mit einem wässerigen Medium, Fermentation des vergärbare Saccharide enthaltenden Extraktes und destillative Aufarbeitung der resultierenden äthanolhältigen Fermentationsflüssigkeit, sowie eine Anlage zur Durchführung des Verfahrens.

Als zuckerhältige Rohstoffe können beispielsweise Zuckerrohr, Zuckerhirse oder Zuckerrüben eingesetzt werden.

Bei Verfahren dieser Art werden zunächst die löslichen Substanzen einschließlich Saccharose und anderer Zucker aus den Rohstoffen durch Extraktion (fälschlich auch "Diffusion" genannt) ausgewaschen.

Zur Extraktion von zerkleinertem Zuckerrohr werden bisher beispielsweise langgestreckte, geringfügig gegen die Horizontale geneigt gelagerte Schneckenförderer verwendet. Die Rohrstücke werden unten aufgebracht und mit am oberen Ende des Schneckenförderers zugeführtem Wasser in Kontakt gebracht. Der Extrakt (Saft) wird vom unteren Ende des Förderers abgezogen.

Nach einer anderen bekannten Technik werden die zerkleinerten Rohstoffe einem mit feinen Öffnungen versehenen Förderband aufgegeben und aus darüber befindlichen Düsen mit scharfen Flüssigkeitsstrahlen hohen Druckes besprüht. Der anfallende Extrakt wird in einem Trog gesammelt.

Der apparative Aufwand und der Energiebedarf für den Extraktionsschritt sind bei den bekannten Methoden hoch.

Der zuckerhältige Extrakt wird - gegebenenfalls nach Kühlung - in einer Fermentationsstation, welche einen oder mehrere Gärbehälter (Fermentoren) umfaßt, nach Zusatz geeigneter Mikroorganismen - beispielsweise Saccharomyces-Stämmen - vergoren.

Die resultierende gärprodukt-(hauptsächlich äthanol-) hältige Fermentationsflüssigkeit wird einer oder mehreren Destillations- bzw. Rektifikationskolonnen zugeleitet, von denen als Kopfprodukt im wesentlichen Äthanol und als Sumpfprodukt die sogenannte Schlempe abgezogen wird.

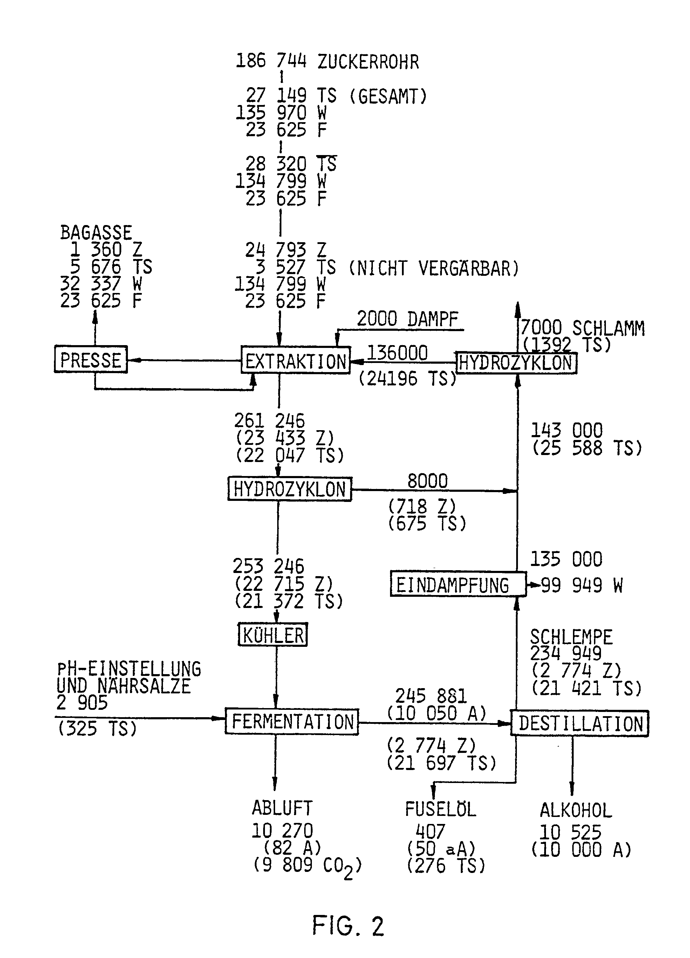

Die DE-A-29 19 518 betrifft ein Verfahren zur Herstellung von Äthanol aus Zuckerrohr, wobei Zuckerrohrschnitzel zwingend 5 bis 45 min lang unter einem Dampfdruck von 0,5 bis 5,0 at gehalten und dann auf ca. 30°C abgekühlt werden. Der Druck im Dämpfer wird mittels Direktdampf eingestellt (Abb. 2 der DE-A).

Vor der Einführung in den Dampfkocher kann den Schnitzeln heiße Schlempe in nicht näher bezeichneter Menge zugesetzt werden. Von einer Rückführung der Schlempe im Gegenstrom zu den Schnitzeln kann daher nicht gesprochen werden.

Nach der DE-A-29 19 518 wird auch keine Extraktion der zuckerhältigen Rohstoffe vorgenommen, sondern die alkoholische Gärung wird durch Zugabe von Hefen direkt in die abgekühlte Mischung aus dem Dampfkocher, also in der Maische mit Bagasse, durchgeführt (vgl. insbesondere Seite 5, 2. Absatz der DE-A). Erst die vergorene Maische wird zur Abtrennung der Feststoffe einer Entwässerungspresse zugeführt.

Die Abtrennung der nicht vergärbaren, löslichen Trockensubstanz erfolgt unter der Annahme einer 100%igen Schlemperückführung (wofür sich allerdings in der DE-A keine konkreten Anhaltspunkte finden) nur über die Bagasse, u.zw. erst nach der Gärung beim Abpressen der Bagasse aus der alkoholischen Maische. Das Verfahren hat den Nachteil, daß die Gärführung aufgrund des hohen Feststoffanteiles in der Maische sehr schwierig ist und kaum Heferückführung angewandt werden kann. Dadurch, daß die Bagasse erst nach der Gärung abgetrennt wird, ist das System von der nicht vergärbaren, löslichen Trockensubstanz nicht effektiv entlastet wie das anmeldungsgemäße Verfahren. Dazu kommt noch, daß nach der DE-A-29 19 518 nur an eine einmalige Rückführung der Schlempe gedacht ist, welche dann ca. 8% gelöste Stoffe enthält und zu organishen Düngemitteln aufgearbeit wird (vgl. Seite 6 der DE-A-29 19 518, Punkt a.)).

Die Schlempe, welche unvergärbare Bestandteile der Rohstoffe, Rückstände der verwendeten Mikroorganismen und Salze enthält, stellt in ständig steigendem Ausmaß ein Entsorgungsproblem ersten Ranges dar. Ein Eindampfen der Schlempe zu wenigstens begrenzt lagerfähigen, trockenen Festprodukten, wie es mancherorts durchgeführt wurde, kommt wegen der hohen Energiekosten heute auch kaum mehr in Frage.

Die Erfindung stellt sich die Aufgabe, die dargelegten Schwierigkeiten und Nachteile, wie sie mit bekannten Verfahren zur Herstellung von Äthanol aus pflanzlichen Rohstoffen verbunden sind, zu überwinden und ein Verfahren sowie eine besonders betriebssichere Anlage zu schaffen, für welche keine komplizierten Apparaturen erforderlich sind und wobei die entstehenden Mengen an auszuschleusenden Abfallstoffen sowie der Energiebedarf vergleichsweise gering gehalten werden können, ohne jedoch Ausbeuteeinbußen hinnehmen zu müssen.

Die gestellte Aufgabe wird bei einem Verfahren der eingangs definierten Art erfindungsgemäß dadurch gelöst, daß wenigstens ein Teil der bei der destillativen Aufarbeitung der Fermentationsflüssigkeit als Rückstand anfallenden wasserreichen Schlempe zur Extraktion im Gegenstrom zu den Rohstoffen rückgeführt wird, wobei in der Schlempe enthaltene, nicht vergärbare Trockensubstanz zusammen mit bei der Extraktion zurückbleibenden festen Rückständen vor der Fermentation dem System entzogen wird.

Beim erfindungsgemäßen Verfahren kann somit auf die Zufuhr frischen Prozeßwassers in die Extraktionsstufe auch ganz verzichtet werden.

Mit der Schlempe wird ein Großteil an nicht vergärbarer Trockensubstanz (TS) wieder zur Extraktion rückgeführt und reichert sich somit zunächst im System an.

Der Gleichgewichtszustand ist erreicht, wenn die Gesamtmenge an beispielsweise mit Bagasse und Hydrozyklonschlamm aus dem Verfahren ausgeschleuster TS gleich ist jener Menge an nicht vergärbarer TS, die mit den Rohstoffen (beispielsweise Zuckerrohr für den Fall, daß Bagasse als Preßrückstand erhalten wird) zugeführt wird, zuzüglich in der Fermentation nicht verwerteter Restmengen an vergärbaren Monosacchariden, welche im Verlauf der destillativen Aufarbeitung und eventueller Eindampfvorgänge in nicht vergärbare TS umgewandelt werden

Nach einer bevorzugten Ausführungsform wird die Gesamtmenge der anfallenden Schlempe mittels Eindampfens reduziert und die reduzierte Menge wird zur Gänze in die Extraktion rückgeführt.

Auf diese Weise gehen die umweltbelastenden Rückstände praktisch vollständig in die nach der Extraktion der zuckerhältigen Rohstoffe verbleibenden Preßrückstände über. Diese Rückstände werden durch Verbrennung beseitigt, der anorganische Anteil (Salze) bleibt dabei in der Asche zurück und kann mit dieser deponiert oder wiedergewonnen werden. Je nach den eingesetzten Rohstoffen reicht die bei der Verbrennung freigesetzte Energie zur vollständigen oder wenigstens teilweisen Deckung des Wärmebedarfes des Verfahrens.

Wenn geringere Anlagenkosten den Vorrang gegenüber Umweltproblemen haben - also bei Kleinanlagen - kann auch lediglich jene Teilmenge der anfallenden Schlempe in die Extraktion rückgeführt werden, welche dort benötigt wird. Die Eindampfung entfällt und der Überschuß an Schlempe wird abgelassen. Auf diese Weise gelangt immer noch ein großer Teil der umweltbelastenden Stoffe in die Preßrückstände.

Der nach der Extraktion abgepreßte Dünnsaft kann in bekannter Weise gleichfalls in die Extraktion rückgeführt werden.

Zweckmäßig werden die zuckerhältigen Rohstoffe verdünnt mit der zwei- bis vierfachen Masse an wässerigem Medium durch die Extraktion geführt.

Mit dieser Maßnahme ist der Vorteil verbunden, daß das Gemisch aus Rohstoffen und wässerigem Medium pumpfähig gemacht wird, so daß keine sonst üblichen aufwendigen mechanischen Fördereinrichtungen in der Extraktionsstufe notwendig sind.

Vorteilhaft wird das bei der Extraktion vorliegende Gemisch von Rohstoffen und wässerigem Medium unter Druck auf eine Temperatur von über 100°C, insbesondere auf eine Temperatur zwischen 105 und 150°C, erhitzt und anschließend rasch auf atmosphärischen Druck entspannt.

Als Folge einer solchen Behandlung wird ein Aufschluß der Rohstoffe durch Zerreißen der Zellstruktur erreicht, wodurch die Zellinhaltsstoffe besser und vollständiger zugänglich gemacht werden.

Für Zuckerrohr ist bereits eine Aufschlußmethode vorgeschlagen worden, bei der aber das vorzerkleinerte Gut an sich, d.h. noch ohne wässeriges Extraktionsmedium, mechanisch mit Druck beaufschlagt und dann plötzlich entspannt wird.

Der erfindungsgemäß vorteilhaft durchzuführende Aufschluß ist in die Extraktion integriert und somit dem Prozeßablauf besser angepaßt. Darüber hinaus wird auf diese Weise ein weiter erhöhter Aufschlußgrad erreicht.

Die bei der Entspannung des Extraktionsgemisches entstehenden Brüdendämpfe werden vorzugsweise zur Vorwärmung dieses Gemisches und/oder zur Eindampfung des Extraktes eingesetzt.

Die Anlage zur Durchführung des erfindungsgemäßen Verfahrens umfaßt eine Extraktionseinrichtung, eine Fermentationsstation und eine Destillationsapparatur und ist erfindungsgemäß dadurch gekennzeichnet,

- daß die Extraktionseinrichtung aus einer Reihe von unter atmosphärischem Druck stehenden Behältern besteht, in deren Innenraum jeweils eine nicht bis zum Boden der Behälter reichende Trennwand vorgesehen ist, wobei der erste Behälter der Reihe über eine Zugabevorrichtung mit zerkleinerten, zuckerhältigen Rohstoffen beschickbar ist,

- daß in den in Förderrichtung der Rohstoffe gesehen letzten Behälter der Reihe eine Zulaufleitung für Schlempe aus der Destillationsapparatur mündet,

- daß der Bodenteil jedes Behälters über eine eine Dickstoffpumpe enthaltende Extraktionsgemischleitung jeweils mit dem nächsten Behälter verbunden ist,

- daß vom Bodenteil des letzten Behälters der Reihe eine mit einer Dickstoffpumpe ausgestattete Leitung zu einer Extrakt-(Saft-)abtrennvorrichtung führt,

- daß die Behälter untereinander jeweils durch eine Überlaufleitung verbunden sind, wobei vom letzten zum ersten Behälter der Reihe ein absteigendes Niveau der Flüssigkeitspegel des in den Behältern befindlichen Extraktionsgemisches vorgesehen ist, und

- daß an den ersten Behälter eine zur Fermentationsstation führende Extrakt-(Saft-)ableitung angeschlossen ist.

Vorzugsweise sind in die Extraktionsgemischleitung zwischen dem ersten und zweiten Behälter der Reihe in Förderrichtung der Rohstoffe gesehen nach der Dickstoffpumpe hintereinander ein Vorwärmer, ein Druckbehälter und ein Entspannungsgefäß eingebaut.

Besonders vorteilhaft ist der Bodenteil des Druckbehälters über ein Steigrohr mit dem auf erhöhtem Niveau angeordneten Entspannungsgefäß verbunden und der Dampfraum des Entspannungsgefäßes ist über eine Brüdenleitung mit dem Vorwärmer verbunden. Für den Fall, daß der im Druckbehälter entsprechend der Höhe bzw. dem inneren Durchmesser des Steigrohres sich einstellende Druck nicht ausreicht, kann vor der Einmündung des Steigrohres in das Entspannungsgefäß zusätzlich ein Reduzierventil vorgesehen sein.

Beim schnellen Strömen des Gemisches von Rohstoffen und Extraktionsmedium durch das Steigrohr, in welchem sich ein Druckgradient einstellt, resultiert ein besonders gründlicher Aufschluß der Rohstoffe.

Nach einer weiteren bevorzugten Ausführungsform der erfindungsgemäßen Anlage sind in der Extrakt-(Saft-) ableitung hintereinander wenigstens ein Wärmeaustauscher, ein Vakuumverdampfer und ein Kühler vorgesehen.

Die Erfindung wird im folgenden anhand der Zeichnung und der Beispiele noch näher erläutert.

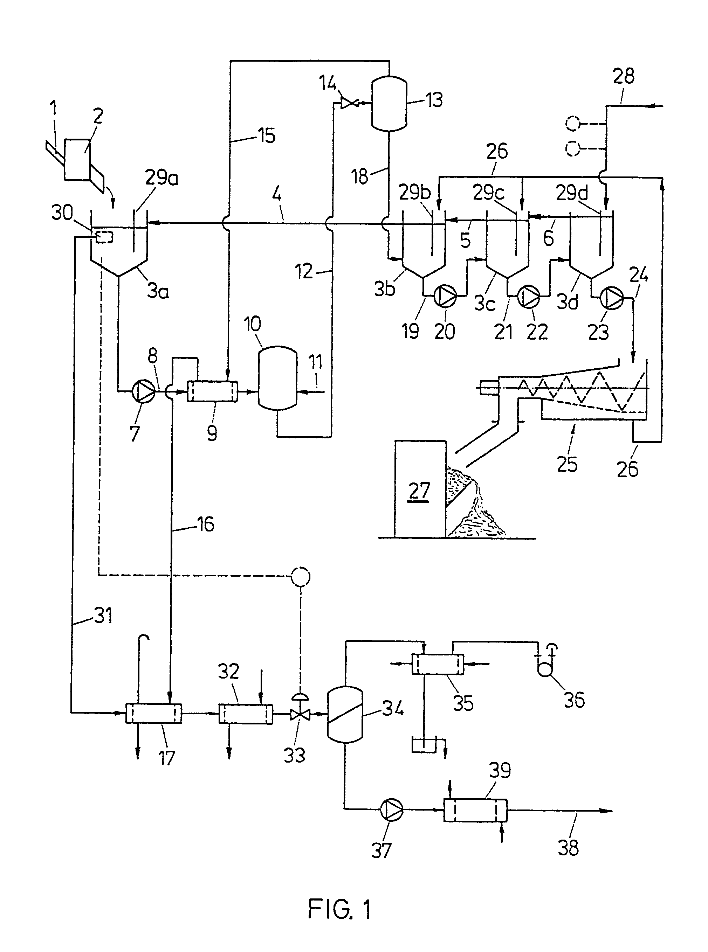

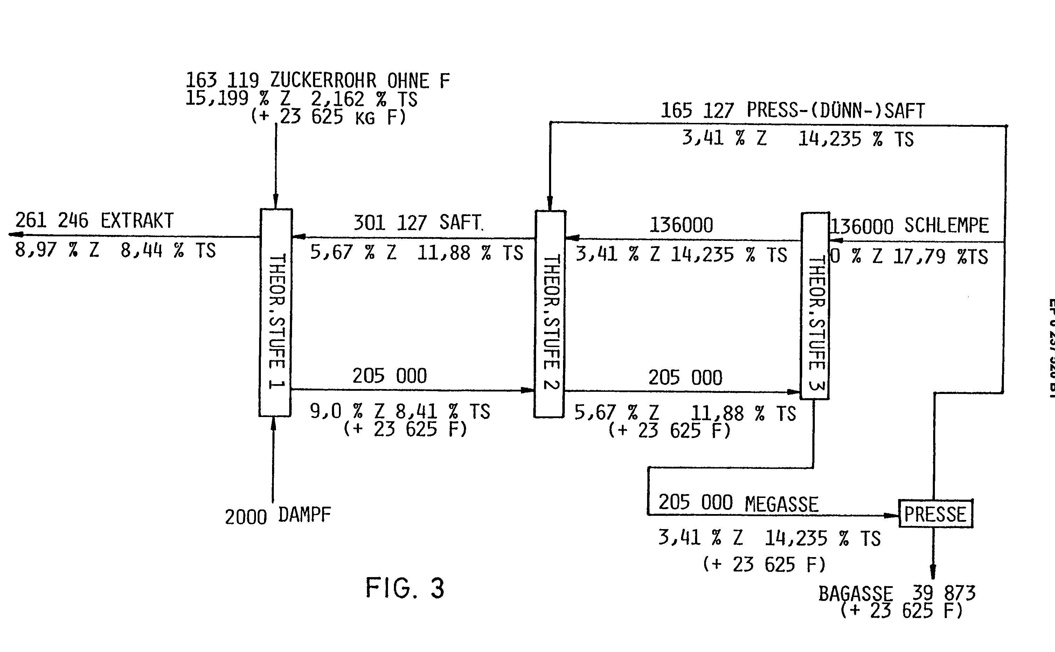

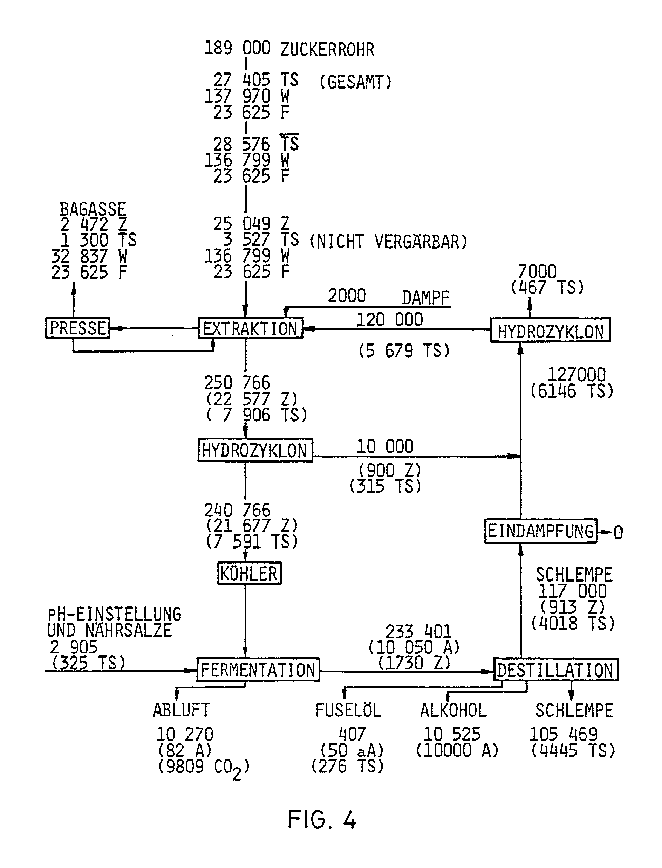

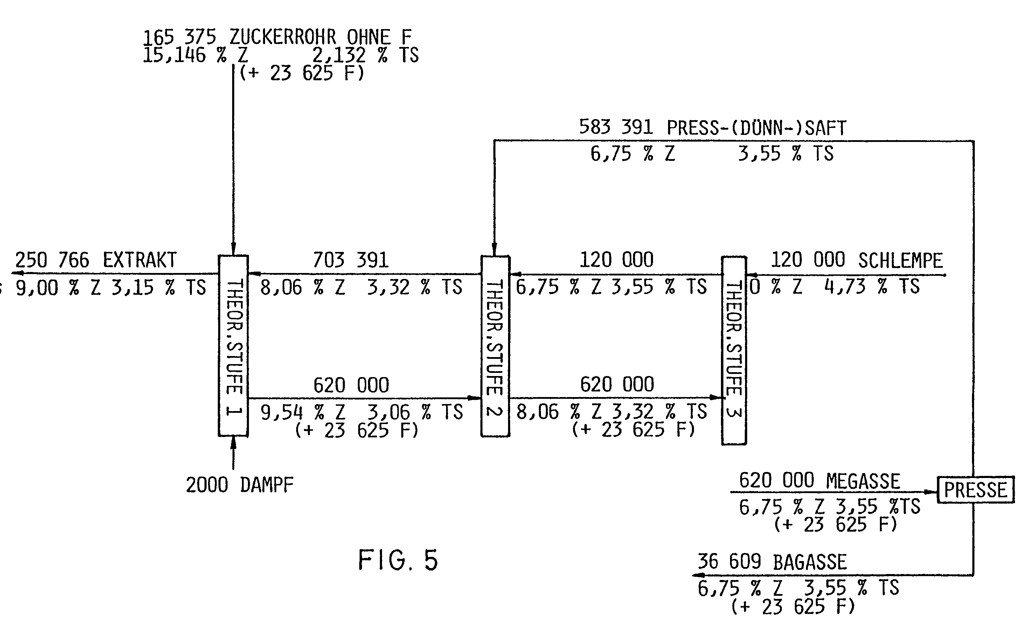

In Fig. 1 ist ein Teil einer erfindungsgemäßen Anlage dargestellt. Fig. 2 zeigt ein Schema einer Ausführungsform des erfindungsgemäßen Verfahrens mit Mengenströmen, aus Fig. 3 ist die Extraktionsstufe gemäß Fig. 2 detaillierter ersichtlich. Fig. 4 veranschaulicht eine andere Ausführungsform des erfindungsgemäßen Verfahrens einschließlich der zugehörigen Mengenströme und in Fig. 5 ist die Gegenstrom-Extraktion mit überlagertem Flüssigkeitskreislauf gemäß Fig. 4 genauer dargestellt.

In Fig. 1 ist mit 1 ein geneigter Zulauftisch bezeichnet, über den die zuckerhältigen Rohstoffe zunächst in eine Zerkleinerungsvorrichtung 2 gelangen. Im Falle des Einsatzes von Zuckerrohr handelt es sich bei der Zerkleinerungsvorrichtung 2 beispielsweise um eine Schneidemaschine, in welcher das Rohr auf ca. 2 cm lange Stücke zerkleinert wird. Die zerkleinerten Rohstoffe fallen in den ersten Behälter 3a, welcher durch die Überlaufleitung 4 mit Extrakt (Saft) aus dem zweiten Behälter 3b beschickt wird. Im dargestellten Beispiel sind insgesamt vier Extraktionsbehälter 3a, 3b, 3c und 3d in Reihe geschaltet, wobei die Überlaufleitungen 4, 5 und 6 in geringfügig gegeneinander versetzten Höhen angeordnet sind, so daß sich vom letzten zum ersten Behälter 3a der Reihe ein absteigendes Niveau der Flüssigkeitspegel des in den Behältern befindlichen Extraktionsgemisches ergibt.

Genauso ist es natürlich auch möglich, die Behälter 3a, 3b, 3c und 3d selbst mit etwas unterschiedlichem Niveau anzuordnen und die Überlaufleitungen an jedem Behälter in gleicher Höhe anzubringen. Der Extrakt bzw. Saft strömt somit in Richtung der eingezeichneten Pfeile vom Behälter 3d zum Behälter 3a.

Vom Bodenteil des Behälters 3a wird das Extraktionsgemisch mit den zerkleinerten Rohstoffen mittels einer Dickstoffpumpe 7 durch die Extraktionsgemischleitung 8 über einen Vorwärmer 9 in einen Druckbehälter 10 gefördert und dort mit Direktdampf aus der Dampfleitung 11 auf erhöhte Temperatur (für Zuckerrohr zweckmäßig auf 110 bis 120°C) gebracht. Durch das Steigrohr 12 gelangt das Extraktionsgemisch anschließend in das Entspannungsgefäß 13.

Knapp vor der Einmündung des Steigrohres 12 in das Entspannungsgefäß 13 ist bei der gezeigten Ausführungsform ein Reduzierventil 14 vorgesehen. Im Entspannungsgefäß 13 entspannt sich das Gemisch auf annähernd Atmosphärendruck. Der Wärmeinhalt der dabei entstehenden Brüdendämpfe wird zur Vorwärmung des Extraktionsgemisches sowie gegebenenfalls zur Vorwärmung des Extraktes (Saftes) aus dem Behälter 3a genützt, sofern der Extrakt mittels Expansionsverdampfung eingeengt werden soll. Zu diesem Zweck ist der Dampfraum des Entspannungsgefäßes 13 über eine Brüdenleitung 15 zunächst mit dem Vorwärmer 9 verbunden, dessen Ableitung 16 mit einem Wärmeaustauscher 17 zum Vorwärmen des Extraktes in Verbindung steht. Aus dem Entspannungsgefäß 13 gelangt das Extraktionsgemisch (die sogenannte Pulpe) durch die Leitung 18 in den Behälter 3b, von dessen Bodenteil es (sie) durch die Extraktionsgemischleitung 19, welch letztere eine Dickstoffpumpe 20 enthält, abgezogen und dem nächsten Behälter 3c zugeführt wird.

Auf die gleiche Weise erfolgt die Weiterförderung durch die Extraktionsgemischleitung 21 mit der Dickstoffpumpe 22 in den Behälter 3d. Vom Bodenteil des letzten Behälters 3d der Reihe wird die Pulpe durch eine mit der Dickstoffpumpe 23 ausgestattete Leitung 24 einer Extrakt-(Saft-) abtrennvorrichtung zugeführt - im dargestellten Fall einer Siebschneckenpresse 25. Der abgepreßte, sogenannte Dünnsaft, fließt durch die Saftleitung 26 wieder in einen der Behälter 3b oder 3c oder auch in beide Behälter zurück, die Preßrückstände, beispielsweise Bagasse, fallen nach der gezeigten Ausführungsform in die Dosiereinrichtung eines Rückstandsverbrennungskessels 27.

In den letzten Behälter 3d der Reihe mündet - von einer nicht gezeigten Destillationsapparatur kommend - eine Zulaufleitung 28 für Schlempe.

Die Behälter 3a, 3b, 3c und 3d sind jeweils mit einer tief in die Flüssigkeit eintauchenden Trennwand 29a, 29b, 29c bzw. 29d ausgestattet, um eine Kurzschlußströmung zu unterbinden und einen intensiven Kontakt des im Gegenstrom zu dem Extraktionsgemisch bzw. der Pulpe geführten Extraktionsmediums (Schlempe und Dünnsaft) zu erzwingen. Ein Teil des Extraktes, d.h. des mit Zuckerstoffen und unvergärbaren Substanzen angereicherten Saftes, wird aus dem ersten Behälter 3a über einen Siebkorb 30 durch eine Extraktableitung 31 abgezogen und dem Wärmeaustauscher 17 zugeführt, aus dem der Extrakt in einen zweiten Wärmetauscher 32 gelangt, in welch letzterem seine Temperatur weiter erhöht wird.

Über ein Regelventil 33 wird der Extrakt weiter in einen Vakuumverdampfer 34 geführt. Der Dampfraum des Vakuumverdampfers 34 ist über einen Kondensator 35 an eine Vakuumpumpe 36 angeschlossen. Der aufkonzentrierte Extrakt wird mit einer Pumpe 37 durch die Leitung 38 einer nicht dargestellten Fermentationsstation zugeführt. Da der aufkonzentrierte Extrakt mit einer Temperatur von etwa 50°C anfällt, kann er im Kühler 39 noch auf die zur Fermentation geeignete Temperatur abgekühlt werden.

Einige der erforderlichen Meß- und Regeleinrichtungen einschließlich der zugehörigen elektrischen Leitungen sind in Fig. 1 strichliert eingezeichnet.

Wird entsprechend wenig Schlempe in den letzten Behälter 3d eingespeist, kann die im Zusammenhang mit der Ausführungsform nach Fig. 1 beschriebene Expansionseindampfung auch entfallen.

Beispiel 1 :

Zur Herstellung von 10.000 kg Äthanol werden als zuckerhältiger Rohstoff 186.744 kg zerkleinertes Zuckerrohr mit einem Gehalt von 72,8% Wasser und 12,65% Fasern eingesetzt. In dem in Fig. 2 dargestellten Verfahrensschema bedeuten sämtliche Zahlenwerte Mengenangaben in kg. Da die Fasern im wesentlichen den Prozeß durchlaufen, ohne die Mengenbilanz zu beeinflussen, sind im folgenden alle Konzentrationsangaben auf den Flüssigkeitsanteil exklusive Fasern bezogen. Die Abkürzungen in den Fig. 2 und 3 bedeuten :

TS : Trockensubstanz
$\overline{TS}$ : Trockensubstanz, wobei der Anteil an vergärbaren Zuckern auf Monosaccharide umgerechnet wurde
W : Wasser
F : Fasern
Z : Vergärbare Monosaccharide
A : Äthanol
aA : andere Alkohole

Die Druckerhitzung des bei der Extraktion vorliegenden Gemisches zum Aufschließen des Rohstoffes erfolgt mit 2.000 kg Wasserdampf.

Bei dem Verfahrensschema wird angenommen, daß der weiter oben erläuterte Gleichgewichtszustand von zu- und abgeführten Stoffen eingestellt ist.

Die Gesamtmenge der anfallenden Schlempe wird mittels Eindampfens reduziert und die reduzierte Menge wird zur Gänze (nach Schlammabtrennung in einem Hydrozyklon) in die Extraktion rückgeführt.

Die Vorgänge bei der Extraktion sind in Fig. 3 detaillierter dargestellt. Zerkleinertes Zuckerrohr mit einem Flüssigkeitsanteil von rund 163 t mit ca. 15,2% Z und ca. 2,16% nicht vergärbarer TS wird im Gegenstrom geführt zu 136 t Schlempe mit ca. 17,8% TS. Als Produkte ergeben sich dabei einerseits ca. 261 t Extrakt (Saft) mit ca. 9% Z und 8,44% TS, andererseits Zuckerrohr-Faserrückstände (Megasse) mit einem Flüssigkeitsanteil von 205 t mit ca. 3,4% Z und ca. 14,2% TS. Die Megasse wird gepreßt. Ca. 165 t gewonnener Preßsaft (Dünnsaft) werden der Extraktion zugeführt. Die verbleibende Bagasse wird üblicherweise in einem Kessel zur Energieversorgung der Anlage verbrannt.

Für eine theoretische Stufe wird angenommen, daß nach intensiver Mischung in der Stufe die ausgehenden Produkte gleiche Konzentration haben. Fig. 3 zeigt, daß knapp drei theoretische Stufen erforderlich sind. Tatsächlich sind natürlich mehr Stufen erforderlich, da die völlige Konzentrationsangleichung in der Praxis nicht ganz erreicht wird.

Die Konzentration des Extraktes an Z bzw. TS kann auch höher eingestellt werden.

Das aus der Eindampfung (siehe Fig. 2) kommende Kondensat (ca. 100 t W) ist nur wenig belastet und stellt kein Entsorgungsproblem dar.

Beispiel 2 :

Zur Herstellung von 10 t Äthanol werden gemäß Fig. 4 ca. 189 t zerkleinertes Zuckerrohr mit 73% Wasser und 12,5% Fasern eingesetzt. Die Abkürzungen haben die gleiche Bedeutung wie in Fig. 2 und 3.

Es wird weitgehend analog zu dem in Fig. 2 gezeigten Fließschema verfahren, mit dem Unterschied, daß keine Eindampfung stattfindet. Nur der in der Extraktion benötigte Anteil an Schlempe wird rückgeführt. Der verbleibende Überschuß an Schlempe wird aus dem Prozeß geschleust. Dabei gelangen immerhin noch ca. 46% der umweltbelastenden Stoffe in die Bagasse, nur der Rest geht mit der Überschußschlempe ab.

Um eine besonders einfache und billige Gestaltung der apparativen Ausrüstung zu ermöglichen, wird das zerkleinerte Zuckerrohr mit einer größeren Menge an wässerigem Medium durch die Extraktion geführt, siehe dazu Fig. 5. Der eingespeisten Menge an zerkleinertem Zuckerrohr wird ca. die dreifache Menge Dünnsaft aus der Presse zugemischt, so daß aus Stufe 1 Zuckerrohrstücke mit einem Flüssigkeitsanteil von 620 t gegenüber eingespeisten 165 t der Stufe 2 zugeführt werden. Dementsprechend werden vor und in der Presse über 583 t Dünnsaft abgeschieden, die im Gegenstrom zu den 620 t Zuckerrohr-Pulpe geführt werden. Anders ausgedrückt, zu den 165 t Zuckerrohr (exklusive Fasern) und 120 t Schlempe, die im Gegenstrom geführt werden, kommen ca. 500 t Saft, welche durch die Extraktion im Kreis gefördert werden.

Das stark verdünnte Extraktionsgemisch ist besonders gut pumpbar und kann in einer in Fig. 1 dargestellten Anlage völlig betriebssicher ohne die bisher erforderlichen aufwendigen Fördereinrichtungen verarbeitet werden.

## Ansprüche

1. Verfahren zur Herstellung von Äthanol aus zuckerhältigen Rohstoffen durch Gegenstrom-Extraktion der Rohstoffe mit einem wässerigen Medium, Fermentation des vergärbare Saccharide enthaltenden Extraktes und destillative Aufarbeitung der resultierenden äthanolhältigen Fermentationsflüssigkeit, dadurch gekennzeichnet, daß wenigstens ein Teil der bei der destillativen Aufarbeitung der Fermentationsflüssigkeit als Rückstand anfallenden wasserreichen Schlempe zur Extraktion rückgeführt wird, wobei in der Schlempe enthaltene, nicht vergärbare Trockensubstanz zusammen mit bei der Extraktion zurückbleibenden festen Rückständen vor der Fermentation dem System entzogen wird

2. Verfahren nach Anspruch 1, dadurch gekennzeichnet, daß die Gesamtmenge der anfallenden Schlempe mittels Eindampfens reduziert wird und die reduzierte Menge zur Gänze in die Extraktion rückgeführt wird.

3. Verfahren nach Anspruch 1 oder 2, dadurch gekennzeichnet, daß die zuckerhältigen Rohstoffe verdünnt mit der zwei- bis vierfachen Masse an wässerigem Medium durch die Extraktion geführt werden.

4. Verfahren nach einem der Ansprüche 1 bis 3, dadurch gekennzeichnet, daß das bei der Extraktion vorliegende Gemisch von Rohstoffen und wässerigem Medium unter Druck auf eine Temperatur von über 100°C erhitzt und anschließend rasch auf atmosphärischen Druck entspannt wird.

5. Verfahren nach Anspruch 4, dadurch gekennzeichnet, daß das Gemisch auf eine Temperatur zwischen 105 und 150°C erhitzt wird.

6. Verfahren nach einem der Ansprüche 4 und 5, dadurch gekennzeichnet, daß die bei der Entspannung des Extraktionsgemisches entstehenden Brüdendämpfe zur Vorwärmung des Gemisches eingesetzt werden.

7. Verfahren nach einem der Ansprüche 4 bis 6, dadurch gekennzeichnet, daß die bei der Entspannung des Extraktionsgemisches entstehenden Brüdendämpfe zur Eindampfung des Extraktes eingesetzt werden.

8. Anlage zur Durchführung des Verfahrens nach einem der Ansprüche 1 bis 3, mit einer Extraktionseinrichtung, einer Fermentationsstation und einer Destillationsapparatur, gemäß Fig. 1, dadurch gekennzeichnet,
– daß die Extraktionseinrichtung aus einer Reihe von unter atmosphärischem Druck stehenden Behältern (3a, 3b, 3c, 3d) besteht, in deren Innenraum jeweils eine nicht bis zum Boden der Behälter reichende Trennwand (29a, 29b, 29c, 29d) vorgesehen ist, wobei der erste Behälter (3a) der Reihe über eine Zugabevorrichtung (2) mit zerkleinerten, zuckerhältigen Rohstoffen beschickbar ist,
– daß in den in Förderrichtung der Rohstoffe gesehen letzten Behälter (3d) der Reihe eine Zulaufleitung (28) für Schlempe aus der Destillationsapparatur mündet,
– daß der Bodenteil jedes Behälters (3a, 3b, 3c) über eine eine Dickstoffpumpe (7, 20, 22) enthaltende Extraktionsgemischleitung (8, 19, 21) jeweils mit dem nächsten Behälter verbunden ist,
– daß vom Bodenteil des letzten Behälters (3d) der Reihe eine mit einer Dickstoffpumpe (23) ausgestattete Leitung (24) zu einer Extrakt-(Saft-) abtrennvorrichtung (25) führt,
– daß die Behälter (3a, 3b, 3c, 3d) untereinander jeweils durch eine Überlaufleitung (4, 5, 6) verbunden sind, wobei vom letzten zum ersten Behälter (3a) der Reihe ein absteigendes Niveau der Flüssigkeitspegel des in den Behältern befindlichen Extraktionsgemisches vorgesehen ist, und
– daß an den ersten Behälter (3a) eine zur Fermentationsstation führende Extrakt-(Saft-) ableitung (31 ; 38) angeschlossen ist.

9. Anlage nach Anspruch 8 zur Durchführung des Verfahrens nach einem der Ansprüche 1 bis 7, dadurch gekennzeichnet, daß in die Extraktionsgemischleitung (8) zwischen dem ersten und zweiten Behälter (3b) der Reihe in Förderrichtung der Rohstoffe gesehen nach der Dickstoffpumpe (7) hintereinander ein Vorwärmer (9), ein Druckbehälter (10) und ein Entspannungsgefäß (13) eingebaut sind.

10. Anlage nach Anspruch 9, dadurch gekennzeichnet, daß der Bodenteil des Druckbehälters (10) über ein Steigrohr (12) mit dem auf erhöhtem Niveau angeordneten Entspannungsgefäß (13) verbunden ist und daß der Dampfraum des Entspannungsgefäßes (13) über eine Brüdenleitung (15) mit dem Vorwärmer (9) verbunden ist.

11. Anlage nach einem der Ansprüche 8 bis 10, dadurch gekennzeichnet, daß in der Extrakt-(Saft-)ableitung (31) hintereinander wenigstens ein Wärmeaustauscher (17 ; 32), ein Vakuumverdampfer (34) und ein Kühler (39) vorgesehen sind.

## Claims

1. Process for the production of ethanol from sugar-containing raw materials by counterflow extraction of the raw materials with an aqueous medium, fermentation of the extract containing fermentable saccharides and distillation-processing of the resulting ethanol-containing fermentation liquid, characterised in that at least part of the water-enriched stillage incurring as residue in the distillation-processing of the fermentation liquid is recycled to extraction, nonfermentable dry substance contained in the stillage being withdrawn from the system prior to fermentation along with solid residues from the extraction.

2. Process according to claim 1, characterised in that the total amount of incurring stillage is reduced by vapourisation and the reduced amount is fully recycled into the extraction.

3. Process according to claim 1 or 2, characterised in that the sugar-containing raw materials are guided through the extraction in a condition diluted by two to four times the mass of aqueous medium.

4. Process according to one of claims 1 to 3, characterised in that the mixture of raw materials and aqueous medium present at the extraction is heated under pressure to a temperature of above 100°C and subsequently quickly released to atmospheric pressure.

5. Process according to claim 4, characterised in that the mixture is heated to a temperature of between 105 and 150°C.

6. Process according to one of claims 4 and 5, characterised in that the exhaust vapours forming during release of the extraction mixture are used for preheating the mixture.

7. Process according to one of claims 4 to 6, characterised in that the exhaust vapours forming during release of the extraction mixture are used for vapourising the extract.

8. Arrangement for carrying out the process according to one of claims 1 to 3, with an extraction means, a fermenting station and a distillation apparatus according to Fig. 1, characterised in
- that the extraction means consists of a series of vessels (3a, 3b, 3c, 3d) under atmospheric pressure, in whose interior space a partition wall (29a, 29b, 29c, 29d) each, not reaching as far as to the bottom of the vessel, is provided, the first vessel (3a) of the series being feedable with comminuted sugar-containing raw material via a charging means (2),
- that a feeding means (28) for stillage coming from the distillation apparatus enters into the last vessel (3d) of the series, seen in conveying direction of the raw materials,
- that the bottom portion of each vessel (3a, 3b, 3c) is each connected with the next vessel via an extraction mixture conduit (8, 19, 21) including thick matter pumping means (7, 20, 22),
- that, from the bottom portion of the last vessel (3d) of the series, a conduit (24) equipped with a thick matter puming means (23) leads to an extract (juice) separating means (25),
- that the vessels (3a, 3b, 3c, 3d) among themselves are each connected by an overflow conduit (4, 5, 6), a descending level of the liquid height of the extraction mixture contained in the vessels being provided from the last to the first vessel (3a) of the series, and
- that an extract (juice) discharge means (31 ; 38) is connected to the first vessel (3a) to lead to the fermenting station.

9. Arrangement according to claim 8 for carrying out the process according to one of claims 1 to 7, characterised in that a preheater (9), a pressure tank (10) and a release tank (13) are consecutively incorporated into the extraction mixture conduit means (8) between the first and the second vessel (3b) of the series, seen in conveying direction of the raw materials, following upon the thick matter pumping means (7).

10. Arrangement according to claim 9, characterised in that the bottom portion of the pressure tank (10) is connected with the release tank (13) arranged at an elevated level via an ascending pipe (12) and that the vapour space of the release tank (13) is connected with the preheater (9) via an exhaust vapour conduit means (15).

11. Arrangement according to one of claims 8 to 10, characterised in that, in the extract (juice) discharge conduit means (31), at least one heat exchanger (17 ; 32), a vacuum evaporator (34) and a cooler (39) are arranged one behind the other.

**Revendications**

1. Procédé pour la préparation d'éthanol à partir de matières premières sacchariféres par extraction à contre-courant des matières premières avec un milieu aqueux, fermentation de l'extrait contenant des saccharides fermentescibles et retraitement par distillation du liquide de fermentation éthanolifère obtenu, caractérisé en ce qu'une partie au moins de la vinasse riche en eau obtenue comme déchet lors du retraitement par distillation du liquide de fermentation est recyclée dans l'extraction, la substance sèche non fermentescible contenue dans la vinasse étant retirée du système avant la fermentation en même temps que les résidus solides restant lors de l'extraction.

2. Procédé selon la revendication 1, caractérisé en ce que la quantité totale de vinasse obtenue est réduite par évaporation et que la quantité réduite est recyclée en totalité dans l'extraction.

3. Procédé selon la revendication 1 ou 2, caractérisé en ce que les matières premières sacchariféres sont passées par l'extraction après dilution avec la masse double à quadruple de milieu aqueux.

4. Procédé selon l'une des revendications 1 à 3, caractérisé en ce que le mélange de matières premières et de milieu aqueux disponible pour l'extraction est chauffé sous pression à une température supérieure à 100°C et est ensuite détendu rapidement à la pression atmosphérique.

5. Procédé selon la revendication 4, caractérisé en ce que le mélange est chauffé à une température comprise entre 105 et 150°C.

6. Procédé selon l'une des revendications 4 et 5, caractérisé en ce que les vapeurs formées lors de la détente du mélange d'extraction sont utilisées pour préchauffer le mélange.

7. Procédé selon l'une des revendications 4 à 6, caractérisé en ce que les vapeurs formées lors de la détente du mélange d'extraction sont utilisées pour concentrer le résidu par évaporation.

8. Installation pour la mise en oeuvre du procédé selon l'une des revendications 1 à 3, comportant un dispositif d'extraction, un poste de fermentation et un appareillage de distillation selon la figure 1, caractérisée en ce que :

    – le dispositif d'extraction comprend une série de récipients sous pression atmosphérique (3a, 3b, 3c, 3d) à l'intérieur desquels est prévue une cloison (29a, 29b, 29c, 29d) n'en atteignant pas le fond, le premier récipient (3a) de la série étant alimentable en matières premières saccharifères broyées par l'intermédiaire d'un dispositif d'apport (2),

    – une conduite d'arrivée (28) pour la vinasse en provenance de l'appareillage de distillation débouche dans le dernier récipient (3d) de la série considéré comme le dernier dans le sens de transport des matières premières,

    – le fond de chaque récipient (3a, 3b, 3c) est relié au récipient suivant par une conduite pour mélange d'extraction (8, 19, 21) comportant une pompe à liquides épais (7, 20, 22),

    – une conduite (24) dotée d'une pompe à liquides épais (23) relie le fond du dernier récipient (3d) de la série à un dispositif de séparation de l'extrait (jus) (25),

    – les récipients (3a, 3b, 3c, 3d) sont reliés entre eux à chaque fois par une conduite de trop-plein (4, 5, 6), un niveau de liquide décroissant étant prévu du dernier au premier récipient (3a) de la série pour le mélange d'extraction se trouvant dans les récipients et

    – une conduite de dérivation pour l'extrait (jus) (31 ; 38) alimentant le poste de fermentation est raccordée au premier récipient (3a).

9. Installation selon la revendication 8 pour la mise en oeuvre du procédé selon l'une des revendicatins 1 à 7, caractérisée en ce que sont incorporés successivement, après la pompe à liquide épais (7), un préchauffeur (9), un réservoir à pression (10) et un réservoir de détente (13) dans la conduite pour liquide d'extraction (8) entre les récipients de la série considérés comme le premier et le second (3b) dans le sens de transport des matières premières.

10. Installation selon la revendication 9, caractérisée en ce que le fond du réservoir à pression (10) est relié par une conduite ascendante (12) au réservoir de détente (13) placé à un niveau plus élevé et que la calandre du réservoir de détente (13) est reliée au préchauffeur (9) par une conduite à vapeurs (15).

11. Installation selon l'une des revendications 8 à 10, caractérisée en ce que sont prévus dans la conduite de dérivation pour l'extrait (jus) (31) successivement au moins un échangeur thermique (17 ; 32), un évaporateur à vide (34) et un refroidisseur (39).

FIG. 1

186 744 ZUCKERROHR

27 149 TS (GESAMT)
135 970 W
23 625 F

28 320 TS
134 799 W
23 625 F

BAGASSE
1 360 Z
5 676 TS
32 337 W
23 625 F

24 793 Z
3 527 TS (NICHT VERGÄRBAR)
134 799 W
23 625 F

2000 DAMPF

7000 SCHLAMM
(1392 TS)

PRESSE ← EXTRAKTION ← 136000 (24196 TS) HYDROZYKLON

261 246
(23 433 Z)
(22 047 TS)

143 000
(25 588 TS)

HYDROZYKLON → 8000 (718 Z) (675 TS)

253 246
(22 715 Z)
(21 372 TS)

135 000
EINDAMPFUNG → 99 949 W

KÜHLER

SCHLEMPE
234 949
(2 774 Z)
(21 421 TS)

PH-EINSTELLUNG
UND NÄHRSALZE
2 905
(325 TS)

245 881
(10 050 A)
(2 774 Z)
(21 697 TS)

FERMENTATION → DESTILLATION

ABLUFT
10 270
(82 A)
(9 809 $CO_2$)

FUSELÖL
407
(50 aA)
(276 TS)

ALKOHOL
10 525
(10 000 A)

FIG. 2

163 119 ZUCKERROHR OHNE F
15,199 % Z   2,162 % TS
(+ 23 625 KG F)

165 127 PRESS-(DÜNN-)SAFT
3,41 % Z    14,235 % TS

261 246 EXTRAKT
8,97 % Z   8,44 % TS

THEOR.STUFE 1

301 127 SAFT
5,67 % Z   11,88 % TS

THEOR.STUFE 2

136000
3,41 % Z 14,235 % TS

THEOR.STUFE 3

136000 SCHLEMPE
0 % Z 17,79 %TS

205 000
9,0 % Z 8,41 % TS
(+ 23 625 F)

205 000
5,67 % Z   11,88 % TS
(+ 23 625 F)

2000 DAMPF

205 000 MEGASSE
3,41 % Z   14,235 % TS
(+ 23 625 F)

PRESSE

BAGASSE  39 873
(+ 23 625 F)

FIG. 3

189 000 ZUCKERROHR

27 405 TS  (GESAMT)
137 970 W
 23 625 F

 28 576 T̄S̄
136 799 W
 23 625 F

BAGASSE
 2 472 Z      25 049 Z
 1 300 TS      3 527 TS (NICHT VERGÄRBAR)
32 837 W     136 799 W
23 625 F      23 625 F

                                    7000
                                   (467 TS)
              2000   DAMPF
              120 000
PRESSE ← EXTRAKTION ←          HYDROZYKLON
              (5 679 TS)

                                   127000
                                  (6146 TS)
250 766
(22 577 Z)
( 7 906 TS)

HYDROZYKLON    10 000
               (900 Z)
               (315 TS)

240 766
(21 677 Z)                    EINDAMPFUNG ─ 0
(7 591 TS)

KÜHLER                         SCHLEMPE
                               117 000
                               (913 Z)
PH-EINSTELLUNG                 (4018 TS)
UND NÄHRSALZE      233 401
2 905            (10 050 A)
(325 TS) →  FERMENTATION  (1730 Z)  DESTILLATION

ABLUFT        FUSELÖL    ALKOHOL    SCHLEMPE

10 270          407      10 525     105 469
(82 A)        (50 aA)   (10000 A)   (4445 TS)
(9809 $CO_2$)  (276 TS)

FIG. 4

12

165 375 ZUCKERROHR OHNE F
15,146 % Z          2,132 % TS
(+ 23 625 F)

583 391 PRESS-(DÜNN-)SAFT
6,75 % Z          3,55 % TS

250 766 EXTRAKT          703 391          120 000          120 000 SCHLEMPE
9,00 % Z 3,15 % TS  8,06 % Z    3,32 % TS  6,75 % Z 3,55 % TS  0 % Z   4,73 % TS

THEOR.STUFE 1          THEOR.STUFE 2          THEOR.STUFE 3

620 000          620 000
9,54 % Z    3,06 % TS  8,06 % Z 3,32 % TS
(+ 23 625 F)          (+ 23 625 F)

2000 DAMPF

620 000 MEGASSE          PRESSE
6,75 % Z 3,55 %TS
(+ 23 625 F)

FIG. 5

36 609 BAGASSE
6,75 % Z   3,55 % TS
(+ 23 625 F)

EP 0 237 520 B1